# EUROPEAN PATENT APPLICATION

(11) **EP 4 835 059 A1**
(43) Date of publication of application: **04.11.2026**
(21) Application number: 25832129.8
(22) Date of filing: 25.06.2025
(51) Int. Cl.: C12N 5/10, C12N 15/62, A61K 35/17, A61P 35/00

(54) **ENGINEERED TIL CELL WITH ENHANCED SUSTAINED KILLING CAPACITY AND USE THEREOF**

(30) Priority: 05.07.2024 CN 202410901374
(71) Applicant: Shanghai Chuangyinsai Biotechnology Co., Ltd., Shanghai 201210 (CN)
(72) Inventor: XU, Aotian, Qingdao, Shandong 266108 (CN); YU, Zhongjie, Qingdao, Shandong 266108 (CN); ZHAO, Yi, Qingdao, Shandong 266108 (CN); GAO, Qing, Qingdao, Shandong 266108 (CN)
(74) Representative: dompatent
(86) International application number: PCT/CN2025/103545
(87) International publication number: WO 2026/007776

(57) **Abstract**

Provided are an engineered TIL cell with an enhanced sustained killing capacity and the use thereof. Specifically, provided is an engineered TIL cell expressing a long-acting activation element, wherein the surface thereof contains a modified DAP10 fusion protein, which can effectively initiate the activation of the TIL cell while reducing the exhaustion caused by the overactivation of TIL. The engineered TIL cell can recognize a tumor cell by means of an NKG2D-NKG2DL pathway, so as to reduce an escape of the tumor cell. In addition, in a culture stage, the engineered cell of the engineered TIL cell has an excellent expansion capacity and cell viability. The engineered TIL cell can efficiently and persistently kill tumor cells, and has application prospects in the field of tumor treatment.

## Description

### Technical field

The present invention relates to the field of biomedicine, and specifically to engineered TIL cell with enhanced sustained killing capacity and use thereof.

### Background

Adoptive cellular immunotherapy (ACI or AIT) refers to the transfer of immune cells with anti-tumor activity, particularly those that have been genetically engineered, into cancer patients, with the aim of directly killing tumor cells or stimulating the body's immune response to eliminate tumor cells. Currently, the most commonly used cellular immunotherapy include CAR-T, TCR-T, and CAR-NK, etc. TIL cells, or tumor-infiltrating lymphocytes, play a crucial role in the body's specific immune response to tumor cells and have distinct advantages over other immune cells.

First, tumor-infiltrating lymphocytes (TILs) can recognize multiple tumor-specific neoantigens and tumor-associated antigens, which allows them to respond more effectively to tumor heterogeneity. Second, TILs typically comprise a certain proportion of effector memory T cells, which express chemokine receptors after being stimulated by tumor antigens *in vivo*, thereby facilitating their enhanced localization to tumor tissues after reinfusion. Furthermore, TILs are derived from the patient's own body and exhibit low toxicity. Therefore, TIL therapy has shown tremendous potential in the field of solid tumors.

However, TIL cells derived from tumor tissue often enter a state of terminal differentiation and exhaustion after activation, resulting in a reduced ability to kill tumor cells. Additionally, a large proportion of TIL cells cultured *in vitro* are non-tumor-specific bystander cells. How to transform bystander cells into effector cells capable of recognizing and killing tumor cells to enhance the efficacy of TIL therapy is one of the challenges yet to be addressed in the field of genetically engineered TIL cells. Moreover, tumor cells frequently escape from immune cell-mediated killing via downregulation of MHC-I molecule expression or TAP gene mutations, leading to loss of antigen presenting function.

Therefore, there is an urgent need in this field to develop engineered TIL cells with highly efficient and long-lasting tumor cell killing capacity.

### Summary of Invention

The aim of the present invention is to provide engineered TIL cells with highly efficient and long-lasting tumor cell killing capacity.

In the first aspect of the present invention, an engineered TIL cell is provided, wherein the engineered TIL cell expresses an exogenous long-acting activation protein, wherein the long-acting activation protein comprises a DAP10 element and a long-acting activation element; and the sequence of the long-acting activation element is set forth in SEQ ID NO: 2.

In another preferred embodiment, the long-acting activation protein comprises, from the N-terminus to the C-terminus, the following structures fused together:
(1) an extracellular domain, comprising a DAP10 element or an active fragment thereof;
(2) a transmembrane domain; and
(3) an intracellular signaling domain, comprising a DAP10 intracellular domain and a long-acting activation element.

In another preferred embodiment, the long-acting activation protein has the structure shown in Formula I:

L-ECD-TM-ICD1-ICD2-ICD3 (Formula I)

in Formula I,
"-" is each independently a peptide bond or a linker peptide;
L is absent or a signal peptide;
ECD is an extracellular domain;
TM is a transmembrane domain;
ICD1 is an intracellular domain derived from DAP10 protein;
ICD2 is absent or a co-stimulation domain;
ICD3 is the long-acting activation element.

In another preferred embodiment, the L is absent.

In another preferred embodiment, the L is a signal peptide from a protein selected from the group consisting of: DAP10, GM-SCF receptor α, CD8, and CD28.

In another preferred embodiment, the amino acid sequence of the L is as set forth in positions 1 to 18 of SEQ ID NO: 1.

In another preferred embodiment, the ECD is an extracellular domain derived from DAP10 protein.

In another preferred embodiment, the amino acid sequence of the ECD is as set forth in positions 19 to 48 of SEQ ID NO: 1.

In another preferred embodiment, the TM is a transmembrane domain derived from DAP10, CD8, or CD28.

In another preferred embodiment, the TM is a transmembrane domain derived from DAP10.

In another preferred embodiment, the amino acid sequence of the TM is as set forth in positions 49 to 69 of SEQ ID NO: 1.

In another preferred embodiment, the ICD1 is an intracellular domain derived from DAP10 protein.

In another preferred embodiment, the sequence of the ICD1 is as set forth in positions 70 to 92 of SEQ ID NO: 1.

In another preferred embodiment, the ICD2 is absent.

In another preferred embodiment, the ICD2 is one, two or more co-stimulation domain(s) derived from protein(s) selected from the group consisting of: CD40, CD27, 4-1BB, OX40, or a combination thereof.

In another preferred embodiment, the ICD2 is a co-stimulation domain derived from CD40 protein.

In another preferred embodiment, the sequence of the ICD2 is as set forth in SEQ ID NO: 15.

In another preferred embodiment, the amino acid sequence of the ICD3 is as set forth in SEQ ID NO: 2.

In another preferred embodiment, the amino acid sequence of the long-acting activation protein is as set forth in SEQ ID NO: 3 or SEQ ID NO: 4.

In another preferred embodiment, the engineered TIL cell comprises the long-acting activation protein on the cell membrane thereof.

In another preferred embodiment, the engineered TIL cell has one or more properties selected from the group consisting of:
(1) long-lasting tumor cell killing capacity;
(2) reduced exhaustion of TIL cell;
(3) reduced tumor evasion and recurrence; and
(4) enhanced *in vivo* amplification capacity.

In another preferred embodiment, the activation is self-activation, which is the engineered TIL cell expressing the long-acting activation protein is activated.

In another preferred embodiment, the activated engineered TIL cell is capable of effectively killing a target cell via NKG2D-NKG2DL interaction.

In another preferred embodiment, the engineered TIL cell comprises a polynucleotide encoding the long-acting activation protein.

In another preferred embodiment, the polynucleotide is DNA, RNA, or a combination thereof.

In another preferred embodiment, the engineered TIL cell comprises a vector comprising a polynucleotide encoding the long-acting activation protein.

In another preferred embodiment, the vector comprises a plasmid and a viral vector.

In another preferred embodiment, the viral vector comprises: a lentiviral vector, an adenoviral vector, or a yellow fever viral vector.

In another preferred embodiment, the vector is a plasmid.

In another preferred embodiment, the engineered TIL cell further expresses a membrane-bound IL-15 fusion polypeptide, which comprises the following elements fused together:
(i) interleukin 15 (IL-15);
(ii) a transmembrane domain of CD86 and an intracellular domain of CD86.

In another preferred embodiment, the amino acid sequence of IL-15 is as set forth in SEQ ID NO: 7.

In another preferred embodiment, the element (i) and element (ii) optionally comprise a linker and/or a hinge region.

In another preferred embodiment, the amino acid sequence of the linker is as set forth in SEQ ID NO: 8.

In another preferred embodiment, the amino acid sequence of the hinge region is as set forth in SEQ ID NO: 9.

In another preferred embodiment, the amino acid sequence of the transmembrane domain and the intracellular domain of CD86 is as set forth in SEQ ID NO: 10.

In another preferred embodiment, the N-terminus of the fusion polypeptide optionally comprises a signal peptide having the amino acid sequence as set forth in SEQ ID NO: 6.

In another preferred embodiment, the engineered TIL cell comprises a nucleic acid construct comprising the polynucleotide encoding the long-acting activation protein.

In another preferred embodiment, the nucleic acid construct comprising a promoter selected from the group consisting of: a constitutive promoter, an inducible promoter, or a combination thereof.

In another preferred embodiment, the promoter is a hypoxia-responsive promoter.

In another preferred embodiment, the hypoxia-responsive promoter is a promoter containing n hypoxia-responsive element(s) (HRE(s)), i.e., a promoter containing n×HRE, where n is an integer selected from 1 to 20.

In another preferred embodiment, n is an integer selected from 2 to 9; more preferably, n is an integer selected from 2 to 4; most preferably, n is 3.

In another preferred embodiment, the hypoxia-responsive promoter is selected from the group consisting of: a TK-mini promoter containing n×HRE element, a CMV-mini promoter containing n×HRE element, and an IL2-mini promoter containing n×HRE element.

In another preferred embodiment, the hypoxia-responsive promoter is a 3×HRE TK-mini promoter.

In another preferred embodiment, the nucleic acid construct further comprise a nucleotide sequence encoding the membrane-bound IL-15 fusion polypeptide.

In another preferred embodiment, the nucleic acid construct further comprises a sequence of a molecular switch element selected from the group consisting of: hEGFRt, BCMA, and CD20.

In another preferred embodiment, the molecular switch element is hEGFRt.

In another preferred embodiment, the nucleotide sequence encoding the long-acting activation protein, the nucleotide sequence encoding the membrane-bound IL-15 fusion polypeptide, and the nucleotide sequence encoding the molecular switch element are linked by a coding sequence of a cleavable linker peptide.

In another preferred embodiment, the cleavable linker peptide is a 2A self-cleaving peptide; more preferably, a T2A peptide.

In another preferred embodiment, the nucleotide sequence encoding the long-acting activation protein, the nucleotide sequence encoding the membrane-bound IL-15 fusion polypeptide, and the nucleotide sequence encoding the molecular switch element are located in separate expression cassettes.

In the second aspect of the present invention, a pharmaceutical composition is provided, which comprises the engineered TIL cell according to the first aspect of the present invention, and a pharmaceutically acceptable carrier.

In the third aspect of the present invention, a kit is provide, which comprises the engineered TIL cell according to the first aspect of the present invention, or an agent used for preparing the engineered TIL cell according to the first aspect of the present invention, wherein the agent is selected from the group consisting of:
(Y1) a polynucleotide encoding the long-acting activation protein; or
(Y2) a vector comprising a polynucleotide encoding the long-acting activation protein.

In the fourth aspect of the present invention, the use of the engineered TIL cell according to the first aspect of the present invention in the manufacture of a medicament for preventing, alleviating and/or treating a tumor is provided.

In another preferred embodiment, the tumor comprises: a solid tumor, a hematologic tumor, or a combination thereof.

In another preferred embodiment, the tumor is selected from the group consisting of: a lung cancer, cervical cancer, gastrointestinal cancer, pancreatic cancer, glioblastoma, ovarian cancer, bladder cancer, liver tumor, breast cancer, colon cancer, rectal cancer, colorectal cancer, endometrial or uterine cancer, salivary gland cancer, kidney cancer, prostate cancer, vulvar cancer, thyroid cancer, liver cancer, anal cancer, penile cancer, testicular cancer, esophageal cancer, bile duct tumor, and head and neck cancer.

In the fifth aspect of the present invention, a method for treating disease is provided, wherein the method comprises administering to a subject in need the cell according to the first aspect of the present invention and/or the pharmaceutical composition according to the second aspect of the present invention.

In another preferred embodiment, the subject is a human or a mammal.

In another preferred embodiment, the disease is tumor or cancer.

In another preferred embodiment, the tumor comprises: a solid tumor, a hematologic tumor, or a combination thereof.

In another preferred embodiment, the tumor is selected from the group consisting of: lung cancer, cervical cancer, gastrointestinal cancer, pancreatic cancer, glioblastoma, ovarian cancer, bladder cancer, liver tumor, breast cancer, colon cancer, rectal cancer, colorectal cancer, endometrial or uterine cancer, salivary gland cancer, kidney cancer, prostate cancer, vulvar cancer, thyroid cancer, liver cancer, anal cancer, penile cancer, testicular cancer, esophageal cancer, bile duct tumor, and head and neck cancer.

It should be understood that, within the scope of the present invention, the above-mentioned technical features of the invention and the technical features specifically described below (e.g., in the Examples) may be combined with each other to constitute new or preferred technical solutions. Due to space limitations, they are not individually enumerated herein.

### Brief Description of the Figures

Figure 1 shows schematic diagrams of the molecular structures of 076, 095, and 096.
Figure 2 shows the results of TIL cells infection positivity rates for each group.
Figure 3 shows the results of TIL cells proliferation assays for each group.
Figure 4 shows cell viability test results for TIL cells from each group after 9 days of expansion culture.
Figure 5 shows the results of IFN-γ secretion assays of TIL cells from each group co-cultured with tumor cells.
Figure 6 shows schematic diagram of the experimental design for assessing the sustained killing capacity of TIL cells against tumor cells.
Figure 7 shows microscopic images of the sustained killing of tumor cells by TIL cells from each group.
Figure 8 shows the results of cytotoxicity assays during the sustained killing of tumor cells by TIL cells from each group.

### Detailed Description

Following extensive and in-depth research, the inventors have, for the first time, provided engineered TIL cells whose cell surfaces express a modified DAP10 fusion protein. The fusion protein contains a long-acting activation element identified through extensive screening, which unexpectedly and effectively initiates TIL cell activation while reducing the exhaustion of TIL caused by excessive activation. The engineered TIL cells of the present invention can recognize tumor cells via the NKG2D-NKG2DL pathway, thereby reducing tumor cell escape and sustaining their activation to initiate killing capacity, thus achieving highly efficient and sustained killing of tumor cells. The present invention was completed on this basis.

### The activation of TIL cells

Natural TIL cells are activated after specifically recognizing tumor cells via MHC-I molecules on the tumor cell surface, thereby acquiring the ability to specifically kill tumor cells. However, among TIL cells obtained through *in vitro* culture, a large proportion are non-tumor-specific bystander cells that cannot be effectively activated. Furthermore, tumor cells often evade TIL-mediated killing by reducing the expression of MHC-I molecules and other mechanisms. In this invention, TIL cells are genetically engineered to enable their activation via the NKG2D-NKG2DL pathway, thereby enhancing their ability to recognize and kill tumor cells and reducing tumor cell escape.

NKG2D is an activating receptor expressed on the surface of NK cells, NKT cells, and CD8+ T cells. It plays a crucial role in innate immunity and is involved in the killing of tumor cells by various immune cells. The NKG2D ligand (NKG2DL) is nearly not expressed in normal cells but is highly expressed on the surface of various tumor cells of different origins, such as colorectal cancer, liver cancer, and brain gliomas. NK and NKT cells can directly recognize and bind to tumor cells via the NKG2D-NKG2DL interaction, thereby killing tumor cells.

However, in TIL cells, DAP10, a downstream molecule of NKG2D, serves only as a co-stimulatory signal. After TIL cells recognize tumor cells via the NKG2D-NKG2DL pathway, they are unable to effectively kill the tumor cells due to the lack of a first signal of activation. Therefore, in this invention, TIL cells are genetically engineered to enable them to recognize and kill tumor cells via the NKG2D-NKG2DL pathway.

TIL cells derived from tumor tissue often enter a state of terminal differentiation and exhaustion after activation, which limits their ability to continuously kill tumor cells. Furthermore, engineered TIL cells developed using existing technologies are prone to becoming overactivated and exhibit significant exhaustion during the killing process, which hinders their ability to persistently and effectively eliminate tumor cells.

The present invention provides long-acting, activated engineered TIL cells that express the long-acting activation protein of the present invention and possess excellent ability to continuously kill tumor cells.

### DAP10

DAP10 exerts its unique intracellular signaling function by binding to cell receptors such as NKG2D. It is highly conserved during evolution, and the DAP10 sequences in mammals (especially primates) exhibit high homology and similar functions. The homology between human and non-human primate DAP10 is as high as 90-100%, and the homology between human and rodent DAP10 is approximately 80%.

The amino acid sequence of human DAP10, with GenBank under accession number AAD46986.1, consists of 92 amino acids, as shown in SEQ ID NO: 1:

MIHLGHILFLLLLPVAAAQTTPGERSSLPAFYPGTSGSCSGCGSLSLPLLA
GLVAADAVASLLIVGAVFLCARPRRSPAQDGKVYINMPGRG (SEQ ID NO:
1).

In the sequence, the signal peptide of DAP10 is as set forth in positions 1 to 18 of SEQ ID NO: 1; the extracellular domain of DAP10 is as set forth in positions 19 to 48 of SEQ ID NO: 1; the transmembrane domain of DAP10 is as set forth in positions 49 to 69 of SEQ ID NO: 1; and the intracellular domain is as set forth in positions 70 to 92 of SEQ ID NO: 1.

In the present invention, DAP10 comprises wild-type DAP10 and mutant DAP10, provided that the mutant DAP10 retains or substantially retains the function of wild-type DAP10, such as retaining ≥50% (preferably ≥60%, ≥70%, ≥80%, or ≥90%) of the function of wild-type DAP10. It should be understood that, in the present invention, DAP10 also comprises mutant proteins whose function exceeds that of wild-type DAP10, such as mutant DAP10 with ≥100% (e.g., 100-200%) of the function of wild-type DAP10. In the present invention, mutations in DAP10 may be naturally occurred or artificially introduced.

### Long-acting activation protein of the present invention

As used herein, the term "long-acting activation protein of the present invention" refers to a fusion protein capable of activating TILs cell that comprises the DAP10 element and the long-acting activation element of the present invention.

In the present invention, "long-acting activation element" refers to a screened sequence with signaling function; its amino acid sequence is as set forth in SEQ ID NO: 2:
GHETGRLSGAADTQALLRNDQVYQPLRDRDDAQYSHLGGNWARNK (SEQ ID NO: 2).

In the present invention, the long-acting activation element further comprises a sequence that has sequence identity with SEQ ID NO: 2, such as a sequence that has ≥85% (e.g., ≥90%, ≥95%, ≥96%, ≥97%, ≥98%, or ≥99%) sequence identity with SEQ ID NO: 2, and has TIL cell activation function identical or similar to that of the sequence set forth in SEQ ID NO: 2.

The long-acting activation protein of the present invention is a membrane-bound fusion protein, comprising DAP10 element and the long-acting activation element, and in particular comprising an extracellular domain, a transmembrane domain, an intracellular domain of DAP10 and the long-acting activation element, and optionally comprising a signal peptide and one or more additional co-stimulation domain(s).

In one embodiment, the long-acting activation protein of the present invention contains signal peptide. The signal peptide is a polypeptide located at the N-terminus of the fusion protein that plays a guiding role in processes such as protein folding, transport, and localization. In the present invention, the choice of signal peptide is not particularly limited, and includes signal peptides commonly used in the art, such as, but not limited to, the DAP10 signal peptide, the GM-CSF receptor α signal peptide, the CD8 signal peptide, the CD28 signal peptide, and the like. In one embodiment, the signal peptide is the DAP10 signal peptide, the sequence of which is as set forth in positions 1 to 18 of SEQ ID NO: 1. In one embodiment, the sequence of the signal peptide is set forth in SEQ ID NO: 12.

In one embodiment, the long-acting activation protein of the present invention does not contain signal peptide. During protein maturation, the signal peptide is typically cleaved off; therefore, the long-acting activation protein that has already been localized to the cell membrane does not contain a signal peptide.

The extracellular domain of the long-acting activation protein of the present invention is the extracellular domain derived from DAP10 protein, preferably, human DAP10. In one embodiment, the sequence of the extracellular domain is as set forth in positions 19 to 48 of SEQ ID NO: 1.

In the present invention, the transmembrane domain of the long-acting activation protein is not particularly limited, preferably, the transmembrane domain is derived from DAP10, CD8 or CD28, more preferably, the transmembrane domain is derived from DAP10. In one embodiment, the sequence of the transmembrane domain is as set forth in positions 49 to 69 of SEQ ID NO: 1.

In the present invention, the intracellular domain of the long-acting activation protein comprises the intracellular domain of DAP10, the long-acting activation element, and optionally an additional co-stimulation domain.

In one embodiment, the long-acting activation protein of the present invention comprises the intracellular domain of DAP10 and the long-acting activation element, and does not contain any additional co-stimulation domain.

In one embodiment, the sequence of the long-acting activation protein without an additional co-stimulation domain is set forth in SEQ ID NO: 3:

MIHLGHILFLLLLPVAAAQTTPGERSSLPAFYPGTSGSCSGCGSLSLPLLA
GLVAADAVASLLIVGAVFLCARPRRSPAQDGKVYINMPGRGGHETGRLSGA
ADTQALLRNDQVYQPLRDRDDAQYSHLGGNWARNK (SEQ ID NO: 3).

In one embodiment, the long-acting activation protein of the present invention comprises the intracellular domain of DAP10, the long-acting activation element, and one or more additional co-stimulation domain(s). Preferably, the additional co-stimulation domain is located between the intracellular domain of DAP10 and the long-acting activation element. The co-stimulation domain is a co-stimulation domain from proteins such as CD40, CD27, 4-1BB and OX40 etc., but is not limited thereto. The co-stimulation domains may be derived from the same protein, or may be derived from different proteins.

In a preferred embodiment, the co-stimulation domain is derived from CD40, and its sequence is set forth in SEQ ID NO: 15.

In one embodiment, the sequence of the long-acting activation protein comprising the co-stimulation domain of CD40 is set forth in SEQ ID NO: 4:

MIHLGHILFLLLLPVAAAQTTPGERSSLPAFYPGTSGSCSGCGSLSLPLLA
GLVAADAVASLLIVGAVFLCARPRRSPAQDGKVYINMPGRGKKVAKKPTNK
APHPKQEPQEINFPDDLPGSNTAAPVQETLHGCQPVTQEDGKESRISVQERQG
HETGRLSGAADTQALLRNDQVYQPLRDRDDAQYSHLGGNWARNK (SEQ ID
NO: 4).

The long-acting activation protein of the present invention can serve as a signaling molecule for the activation of TIL cells. Upon recognition of tumor cells by TIL cells, it efficiently and sustainably activates the TIL cells, thereby enabling them to effectively kill the tumor cells. In addition, the long-acting activation protein of the present invention may also serve as an activation component for other immune cells, such as, but not limited to, T cells and NK cells derived from PBMC, and the like.

### Nucleic acid construct of the present invention

The present invention provides a nucleic acid construct expressing the long-acting activation protein of the present invention. The first expression cassette of the nucleic acid construct of the present invention comprising the sequence encoding the long-acting activation protein of the present invention, and optionally a hypoxia-responsive promoter.

The hypoxia-responsive promoter of the present invention is a promoter containing n hypoxia-responsive element(s) (HRE(s)), i.e., a promoter containing n×HRE. Hypoxia is a hallmark feature of solid tumors and this unique environmental signal can be exploited for targeted cancer therapy, enabling the target gene to be highly expressed in the tumor microenvironment while remaining unexpressed or lowly expressed in normal tissues, thereby maximizing the function of the exogenous gene while minimizing potential side effects. In the construction of a nucleic acid molecule for expressing the fusion protein of the present invention, a hypoxia-responsive promoter is employed to express the fusion protein under hypoxic conditions. The type of promoter used to construct the hypoxia-responsive promoter of the present invention is not particularly limited; preferably, a TK mini promoter containing n×HRE is used.

Preferably, a 3×HRE-TK-mini promoter is used in the present invention.

In one embodiment, the nucleic acid construct of the present invention further comprises a molecular switch element (also known as an immune brake element). When cells expressing a nucleic acid construct present a safety risk, the molecular switch element can serve as a target, allowing the risky cells to be eliminated using corresponding drugs.

In one embodiment, the molecular switch element or immune brake element of the present invention is hEGFRt. When cells comprising the nucleic acid construct of the present invention present a safety risk, the therapeutic anti-hEGFRt monoclonal antibody cetuximab can be administered to eliminate the target cells through ADCC and CDC, thereby further improving safety; meanwhile, flow cytometric detection of the hEGFRt molecule can also be used to indicate the positive rate of cells integrated with the target gene. The exemplary sequence of the molecular switch element is set forth in SEQ ID NO: 16.

In the nucleic acid construct of the present invention, preferably, it contains coding sequence expressing membrane-bound IL-15 (mIL-15), i.e., fusion protein comprising IL-15, transmembrane domain and intracellular domain. In another preferred embodiment, the membrane-bound IL-15 may also be linked to the first expression cassette via a cleavable linker peptide.

Interleukin 15 (IL-15) is a pro-survival cytokine that maintains the homeostasis of long-lived CD8+ memory T cells, inhibits activation-induced cell death (AICD), enhances anti-tumor activity *in vivo*, and reverses T cell exhaustion. Native IL-15 is a small, unstable protein, with a short serum half-life; supraphysiological doses are required to elicit an *in vivo* response. By fusing IL-15 with a transmembrane and an intracellular domain to obtain a membrane-bound IL-15, which has the function of maintaining long-term persistence of the memory stem cell phenotype. Preferably, the intracellular domain is CD86 intracellular domain. Preferably, the transmembrane domain is CD86 transmembrane domain.

In one embodiment, the molecular switch element and the membrane-bound IL-15 element may be linked to the first expression cassette or to each other via a cleavable linker peptide, respectively. Preferably, the cleavable linker peptide is a self-cleaving peptide. Preferably, the self-cleaving peptide is selected from the group consisting of: a T2A peptide, a P2A peptide, and a combination thereof.

In one embodiment, the molecular switch element and the membrane-bound IL-15 element are located in separate expression cassettes and are driven by separate promoters. The promoters are independently constitutive promoter or inducible promoter.

### Vector of the present invention

In the present invention, the term "vector" generally refers to a nucleic acid molecule capable of transporting another nucleic acid to which it is linked. One type of vector is a "plasmid", which refers to a circular double-stranded DNA loop into which other DNA segments can be ligated. Another type of vector is a viral vector, wherein other DNA segments can be ligated into the viral genome.

Certain vectors are capable of self-replication in a host cell into which they have been introduced (e.g., bacterial vectors having a bacterial origin of replication and episomal mammalian vectors). Other vectors (e.g., non-episomal mammalian vectors) can integrate into the genome of the host cell upon introduction into the host cell, and are thereby replicated together with the host genome, such as non-self-replicating naked RNA polynucleotides, naked DNA polynucleotides, polynucleotides composed of DNA and RNA in the same strand, poly-lysine-conjugated DNA or RNA, peptide-conjugated DNA or RNA, liposome-conjugated DNA, and the like.

Furthermore, certain vectors are capable of directing the expression of genes operably linked thereto. Such vectors are referred to in the present invention as "recombinant expression vectors" (or simply "expression vectors"). Generally, expression vectors used in recombinant DNA techniques are in the form of plasmids. In the present specification, "plasmid" and "vector" are used interchangeably, as plasmids are the most commonly used form of vectors.

As used herein, "the vector of the present invention" refers to the vector comprising the nucleic acid construct of the present invention. Preferably, the vector of the present invention is plasmid.

### The engineered TIL cells of the present invention

As used herein, the term "engineered TIL cells of the present invention" and "TIL cells of the present invention" are used interchangeably, and refer to the TIL cells capable of expressing the long-acting activation protein of the present invention and/or containing the vector of the present invention.

The engineered TIL cells of the present invention can recognize tumor cells via the NKG2D-NKG2DL pathway, thereby reducing tumor cell escape, sustaining their activation to initiate killing capacity, and rarely resulting in cell exhaustion, thus achieving highly efficient and sustained killing of tumor cells.

Furthermore, the engineered TIL cells of the present invention exhibit excellent expansion capacity and cell viability during the culture phase, ensuring the longevity of the TIL cells during the killing phase, and are suitable for industrial-scale production.

In one embodiment, the engineered TIL cells further express the membrane-bound IL-15 of the present invention.

In one embodiment, the engineered TIL cells of the present invention further express the molecular switch element of the present invention. When the engineered TIL cells of the present invention present a safety risk, the molecular switch element can serve as a target, allowing the risky cells to be eliminated using corresponding drugs. The molecular switch element of the present invention is selected from the group consisting of group consisted of: hEGFRt, BCMA, CD20. Preferably, the molecular switch element of the present invention is hEGFRt.

### Pharmaceutical composition of the present invention

The pharmaceutical composition of the present invention may comprise the fusion protein of the present invention, or immune effector cells of the present invention (e.g., the engineered TIL cells of the present), and one or more pharmaceutically acceptable carriers, diluents, excipients, and adjuvants. These compositions may be suitable for use in the treatment of the therapeutic diseases described in this article.

In the present invention, the term "subject" may refer to a mammal requiring treatment, such as a human or a veterinary patient (e.g., rodents (such as mice or rats), cats, dogs, dairy cows, horses, sheep, goats, or other livestock). In some embodiments, a "subject" may be a clinical patient, a clinical trial volunteer, a laboratory animal, and the like. The subject may be suspected of having a disease characterized by cell proliferation, or may have developed a disease characterized by cell proliferation, or may have been diagnosed with a disease characterized by cell proliferation; or they may be control subjects confirmed not to have a disease characterized by cell proliferation. As described herein, diagnostic methods for diseases characterized by cell proliferation and the clinical classification of such diagnoses are known to those skilled in the art.

The pharmaceutical composition of the present invention may be used to treat tumors. In the present invention, the terms "tumor" and "tumor cells" generally refer to or describe a physiological condition in mammals typically characterized by unregulated cell growth. Examples of tumors include, but are not limited to, carcinomas, lymphomas, blastomas, sarcomas, neuroendocrine tumors, mesotheliomas, schwannomas, meningiomas, adenocarcinomas, and melanomas. "Tumor cells" may further comprise "solid tumors", which refer to tumors selected from the group consisting of: gastrointestinal cancer, pancreatic cancer, glioblastoma, cervical cancer, ovarian cancer, bladder cancer, liver cancer, breast cancer, colon cancer, rectal cancer, endometrial or uterine cancer, salivary gland cancer, kidney cancer, prostate cancer, vulvar cancer, thyroid cancer, anal cancer, penile cancer, testicular cancer, esophageal cancer, bile duct tumor, and head and neck cancer.

### The main advantages of the present invention include:

(1) The engineered TIL cells of the present invention can recognize and kill tumor cells via the NKG2D-NKG2DL pathway, thereby reducing the escape of tumor cells lacking expression MHC class I molecules.
(2) The engineered TIL cells of the present invention, upon recognition of tumor cells, can be efficiently and persistently activated with reduced TIL cells exhaustion, and possess a sustained, multi-round tumor-killing capacity.
(3) The engineered TIL cells of the present invention exhibit excellent expansion capacity and cell viability during the culture phase.
(4) The engineered TIL cells of the present invention can be induced to express the long-acting activation protein of the present invention under the hypoxic conditions characteristic of solid tumors using a hypoxia-responsive promoter.
(5) The engineered TIL cells of the present invention can further express an immune molecular switch, such as hEGFRt, thereby avoiding potential toxicity with the sustained expression of exogenous proteins and enhancing the safety in clinical treatment.

The present invention will be further described below with reference to specific embodiments. It should be understood that these examples are provided merely to illustrate the invention and are not intended to limit its scope. Experimental methods in the following examples for which specific conditions are not specified are generally carried out under conventional conditions, such as those described in Sambrook et al., Molecular Cloning: A Laboratory Manual (New York: Cold Spring Harbor Laboratory Press, 1989), or as recommended by the manufacturer. Unless otherwise specified, percentages and fractions refer to weight percentages and weight fractions.

### Example 1:The Preparation of TIL Cells Containing the Target Genes

### 1. The construction of plasmids

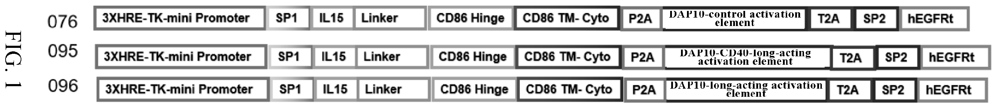

CD3ζ is a commonly used signaling sequence in this field; therefore, in the present invention, CD3ζ is used as a control activation element. As shown in Figure 1, three constructs designated 076, 095, and 096 were generated. Figure 1 panel A shows a simplified diagram of the plasmid structures of 076, 095, and 096, wherein 3×HRE-TK-mini Promoter is a hypoxia-inducible promoter; SP1 is the IgE signal peptide; CD86Hinge is the hinge region of CD86; CD86TM-cyto is the transmembrane and intracellular regions of CD86; T2A and P2A are self-cleaving polypeptide, respectively; and hEGFRt is the third and fourth domains of human EGFR. The sequences of each element are shown in Table 1 below.

**Table 1 The nucleotide or amino acid sequences of each element**

| **Element** | **Sequence** | **SEQ ID NO:** |
|---|---|---|
| The nucleotide sequence of 3×HRE-TK-mini promoter | GCTAACATAACCCGGGAGGTACCGAGCTCTGTCACGTCCTG CACGACTCTAGTTGTCACGTCCTGCACGACTCTAGTTGTCAC GTCCTGCACGACGCTAGCTCGAGATCCGGCCCCGCCCAGCG TCTTGTCATTGGCGAATTCGAACACGCAGATGCAGTCGGGG CGGCGCGGTCCGAGGTCCACTTCGCATATTAAGGTGACGCG TGTGGCCTCGAACACCGAGCGACCCTGCAGCGACCCGCTTA A | 5 |
| The amino acid sequence of signal peptide SP1 (IgE signal peptide) | MDWTWILFLVAAATRVHS | 6 |
| The amino acid sequence of IL-15 | NWVNVISDLKKIEDLIQSMHIDATLYTESDVHPSCKVTAMKCF LLELQVISLESGDASIHDTVENLIILANNSLSSNGNVTESGCKEC EELEEKNIKEFLQSFVHIVQMFINTS | 7 |
| The amino acid sequence of LINKER | SGGGSGGGGSGGGGSGGGGSGGGS | 8 |
| The amino acid sequence of CD86 hinge region | LEDPQPPPDHIP | 9 |
| The amino acid sequence of the transmembrane and intracellular domains of CD86 (CD86TM-Cyto) | WITAVLPTVIICVMVFCLILWKWKKKKRPRNSYKCGTNTMERE ESEQTKKREKIHIPERSDEAQRVFKSSKTSSCDKSDTCF | 10 |
| The amino acid sequence of T2A peptide | EGRGSLLTCGDVEENPGP | 11 |
| The amino acid sequence of signal peptide SP2 (GM-SCF receptor α signal peptide) | MLLLVTSLLLCELPHPAFLLIP | 12 |
| The amino acid sequence of DAP10 | MIHLGHILFLLLLPVAAAQTTPGERSSLPAFYPGTSGSCSGCGSL SLPLLAGLVAADAVASLLIVGAVFLCARPRRSPAQDGKVYINM PGRG | 1 |
| The amino acid sequence of DAP10-control activation element | MIHLGHILFLLLLPVAAAQTTPGERSSLPAFYPGTSGSCSGCGSL SLPLLAGLVAADAVASLLIVGAVFLCARPRRSPAQDGKVYINM PGRGMRVKFSRSADAPAYQQGQNQLYNELNLGRREEYDVLDK RRGRDPEMGGKPQRRKNPQEGLYNELQKDKMAEAYSEIGMK GERRRGKGHDGLYQGLSTATKDTYDALHMQALPPR | 13 |
| The amino acid sequence of P2A peptide | ATNFSLLKQAGDVEENPGP | 14 |
| The amino acid sequence of long-acting activation element | GHETGRLSGAADTQALLRNDQVYQPLRDRDDAQYSHLGGNW ARNK | 2 |
| The amino acid sequence of DAP10-long-acting activation element | MIHLGHILFLLLLPVAAAQTTPGERSSLPAFYPGTSGSCSGCGSL SLPLLAGLVAADAVASLLIVGAVFLCARPRRSPAQDGKVYINM PGRGGHETGRLSGAADTQALLRNDQVYQPLRDRDDAQYSHLG GNWARNK | 3 |
| The co-stimulation sequence of CD40 | KKVAKKPTNKAPHPKQEPQEINFPDDLPGSNTAAPVQETLHGC QPVTQEDGKESRISVQERQ | 15 |
| The amino acid sequence of DAP10-CD40 co-stimulation-long -acting activation element | MIHLGHILFLLLLPVAAAQTTPGERSSLPAFYPGTSGSCSGCGSL SLPLLAGLVAADAVASLLIVGAVFLCARPRRSPAQDGKVYINM PGRGKKVAKKPTNKAPHPKQEPQEINFPDDLPGSNTAAPVQET LHGCQPVTQEDGKESRISVQERQGHETGRLSGAADTQALLRND QVYQPLRDRDDAQYSHLGGNWARNK | 4 |
| The amino acid sequence of hEGFRt | RKVCNGIGIGEFKDSLSINATNIKHFKNCTSISGDLHILPVAFRG DSFTHTPPLDPQELDILKTVKEITGFLLIQAWPENRTDLHAFENL EIIRGRTKQHGQFSLAVVSLNITSLGLRSLKEISDGDVIISGNKNL CYANTINWKKLFGTSGQKTKIISNRGENSCKATGQVCHALCSP EGCWGPEPRDCVSCRNVSRGRECVDKCNLLEGEPREFVENSEC IQCHPECLPQAMNITCTGRGPDNCIQCAHYIDGPHCVKTCPAGV MGENNTLVWKYADAGHVCHLCHPNCTYGCTGPGLEGCPTNG PKIPSIATGMVGALLLLLVVALGIGLFM | 16 |

The molecular sequences of the constructs were synthesized by GenScript Corporation. The synthesized gene sequences were cloned into the basic vector pLV-EF1α-c-MYC-IRES-EGFP (obtained from Wuhan Miaoling), and plasmids 076, 095, and 096 were obtained. Transformation was performed using a kit (TransGen Biotech, CD301), and plasmid extraction was carried out using a kit (Jianshi Bio, TD429).

### 2. Lentivirus preparation and titer assay

The plasmid vectors 076, 095, 096, psPAX2, and pMD2.G obtained from the above steps were separately subjected to large-scale purification. The purified plasmids were mixed at a ratio and transfected into HEK-293T cells (cultured in 100 mm dishes) grown to 70-90% (approximately 80%) confluency using Lipofectamine 3000. At 4 hours post-transfection, the cells were taken out, the supernatant was discarded, and 10 mL of DMEM complete medium was added to each dish; the cells were then placed in a 37°C, 5% CO₂ incubator for further culture. After 48 hours of culture, the culture medium was collected and centrifuged at 400×g for 5 min to retain the supernatant. The supernatant was filtered through a 0.45-µm filter, and the resulting filtrate was the crude recombinant lentivirus solution. The lentivirus was concentrated using an Utra-15 centrifugal filter device, and centrifuged at 5000 rpm for 50 min.

Cell counting was performed on HEK-293T cells that have been washed, trypsin-digested, and serially diluted. After infection, the HEK-293T cells are subjected to hypoxia treatment, and the proportion of cells positive for transgene expression is determined by flow cytometry, and the viral titer is calculated. The viral titer is calculated according to the formula: viral titer = (m×2.5×10⁵×dilution factor) / volume of infection, wherein m is the proportion of cells positive for transgene expression.

### 3. Lentiviral infection of TIL cells

TIL cells were isolated and cultured from tumor masses. According to the viral titers, TIL cells were infected with lentivirus at an MOI of 10. Lentivirus, Lentiboost (100×), and TIL cells (3.5×10⁵) were added to REP medium in centrifuge tube, with each containing 200 µL of medium-virus-cell mixture. After being mixed, the mixture was transferred to a 48-well plate and cultured in a 37°C, 5% CO₂ incubator. At 24 hours post-infection, the cells were collected, centrifuged at 1000 rpm, and the viral supernatant was discarded. An appropriate volume of REP complete medium (AIM V medium : A1640 medium = 1:1) was added to resuspend the cells, which were then transferred to a 24-well plate and cultured in a 37°C, 5% CO₂ incubator. After 72 hours of culture, the cells were collected.

The TIL cells infected with lentivirus containing constructs 076, 095, and 096 were designated as 076TIL, 095TIL, and 096TIL, respectively, and the uninfected control TIL cells were designated as TIL-MOCK.

### 4. Hypoxia treatment of TIL cells

5×10⁵ cells obtained from step 3 were transferred to a new 24-well plate, and then placed in a hypoxia chamber (oxygen concentration of 1%) for hypoxia treatment (experimental group); the remaining TIL cells (serving as a non-hypoxic control group) are transferred to a new 24-well plate and cultured in a 37°C, 5% CO₂ incubator (oxygen concentration of 21%).

### Example 2: The Detection of TIL Positivity Rate

Flow cytometric analysis using indirect antibody staining was performed to determine the positivity rate of TIL cells in each group. 5×10⁵ cells from the experimental group and the control group were each subjected to flow cytometry. The cells in each group were divided into two portions: an all-negative group and a test group, with each portion having a cell volume of 50 µL. IL-15 antibody (Beijing Bio-New Biotechnology Co., Ltd., A09D21-9E) was added to the test group and incubated at room temperature for 15 min. After incubation, 1 mL of PBS containing 2% FBS was added, and the cells were centrifuged at 400×g for 5 min. Then, 50 µL of FITC Donkey anti-rabbit IgG Antibody (BioLegend, 406403) prepared in PBS containing 2% FBS was added to the test group cells and incubated at room temperature for 10 min. Next, 1 mL of PBS containing 2% FBS was added and the cells were centrifuged at 400×g for 5 min. The supernatant was discarded, 50 µL of 7-AAD solution prepared in PBS containing 2% FBS was added to the test group cells and incubated at room temperature for 7 min. Then, 1 mL of PBS containing 2% FBS was added to both the all-negative group and the test group, and the cells were centrifuged at 400×g for 5 min. The supernatant was discarded, the cells were resuspended in 200 µL of PBS containing 2% FBS and subjected to flow cytometric analysis.

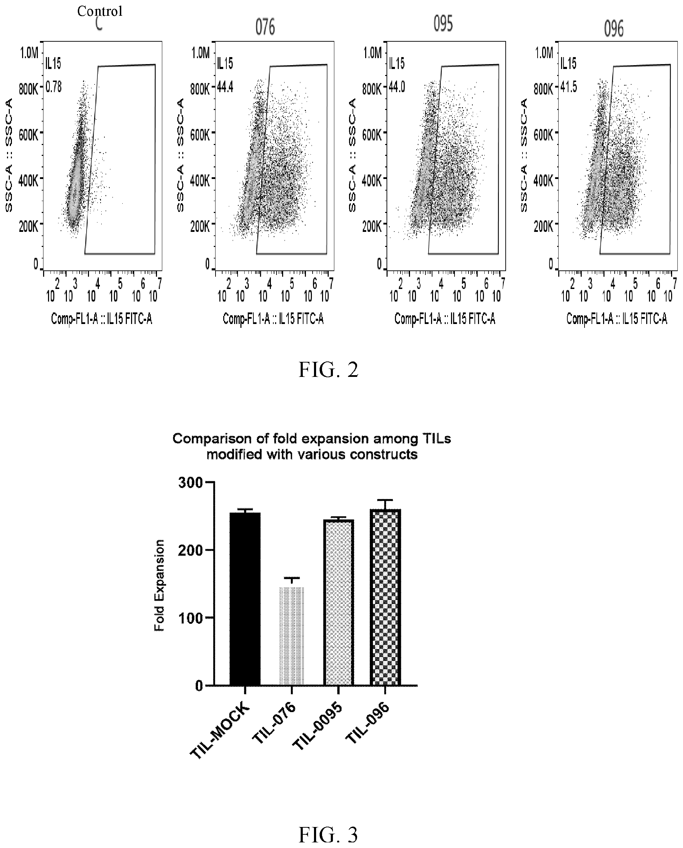

The results of the proportion of mIL-15 detected by flow cytometry are shown in Figure 2. After infection of TIL cells with 076, 095, and 096 lentiviruses, respectively, IL15+ cells were detected by flow cytometry after 4 days of culture. The flow cytometry data showed that the positive rates for 076, 095, and 096 were 44.4%, 44.0%, and 41.5%, respectively, all indicating high-level positive infection rates.

### Example 3: The Assays of TIL Expansion Capacity and Cell Viability

After lentiviral infection, TIL cells were co-cultured with feeder cells (IL-21 NK cell expansion reagent, Zhongying Biotechnology, Cat. No. ZY-NKZ-0104) at a ratio of 1:25, and the cell density was adjusted to 5×10⁵ cells/mL. The cells were cultured in REP medium in 6-well plates with 2 mL per well. Cell counting was performed every two days, and fresh medium was replenished to adjust the cell density to 5×10⁵ cells/mL. After continuous culture for 9 days, cell counting and viability assay were carried out. Each experiment was performed in triplicate.

The fold expansions of TIL-MOCK, 076TIL, 095TIL, and 096TIL are shown in Figure 3, and were 255-, 150-, 245-, and 260-fold, respectively. The fold expansions of 095TIL, 096TIL, and the control TIL were essentially consistent, while the fold expansion of 076TIL was significantly reduced.

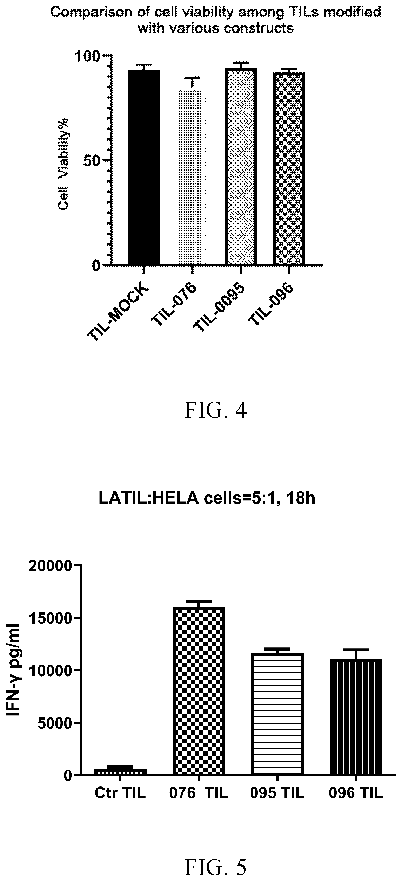

The cell viabilities of TIL-MOCK, 076TIL, 095TIL, and 096TIL after 9 days of expansion culture are shown in Figure 4, and were 93%, 85%, 94%, and 92%, respectively. The cell viabilities of 095TIL, 096TIL, and the control TIL were comparable, whereas the cell viability of 076TIL was decreased.

The above results indicate that, with respect to both fold expansion and cell viability of TIL cells in each group, the 076TIL cells containing the control activation element were lower than those of the control group, whereas the 095TIL and 096TIL cells containing the long-acting activation element were comparable to the control TIL, suggesting that the long-acting activation element is more favorable for the proliferation of the engineered TIL cells.

### Example 4: The Assays of TIL IFN-γ Secretion

The amount of IFN-γ secreted by TIL cells in each group after co-culture with tumor cells was measured. TIL cells were co-cultured with HeLa cells (which highly express NKG2DL) at a ratio of 5:1 (TIL:tumor cells) for 18 hours. The supernatant was collected and detected using an IFN-γ ELISA detection kit. The assay was performed with the Human IFN-γ Precoated ELISA Kit (Dakewe, 2307-3), and the procedures were carried out according to the kit manufacturer's instructions, specifically as follows:
Diluted Cytokine standard was added to the standard wells at 100 µL per well. Samples were diluted with Dilution buffer R (1×) and added to the sample wells at 100 µL per well. The plate was sealed with a sealing film and incubated at room temperature for 2 hours. The liquid in the wells was then discarded, and 1× Washing buffer working solution was added at 300 µL per well. After 1 minute of standing, the liquid was discarded. This washing step was repeated 3 times, and the plate was blotted dry on filter paper each time. Biotinylated antibody working solution was added at 100 µL per well, the plate was sealed with a sealing film, and incubated at room temperature for 1 hour. Streptavidin-HRP working solution was added at 100 µL per well, and the plate was sealed and incubated at room temperature (18-25°C) for 30 minutes. TMB was added at 100 µL per well, and the plate was incubated at room temperature in the dark for 5-30 minutes; the stop reaction was determined according to the depth of color (dark blue) in the wells. Typically, color development for 10-20 minutes can achieve satisfactory results. Stop solution was immediately added at 100 µL per well to terminate the reaction. The OD value was read at 450 nm using a microplate reader. Data were analyzed using a four-parameter logistic method.

The ELISA results are shown in Figure 5. After co-cultured with various tumor cell lines, the IFN-γ secretion levels of 076TIL, 095TIL, and 096TIL were all significantly higher than those of the uninfected TIL-MOCK.

The above results indicate that, compared with control cells, 076TIL, 095TIL, and 096TIL all exhibit significant IFN-γ secretion, suggesting that both the control activation element and the long-acting activation element of the present invention are capable of activating TIL cells via the NKG2D-NKG2L recognition pathway.

### Example 5: The Sustained Killing Capacity of TILs against Tumor Cells

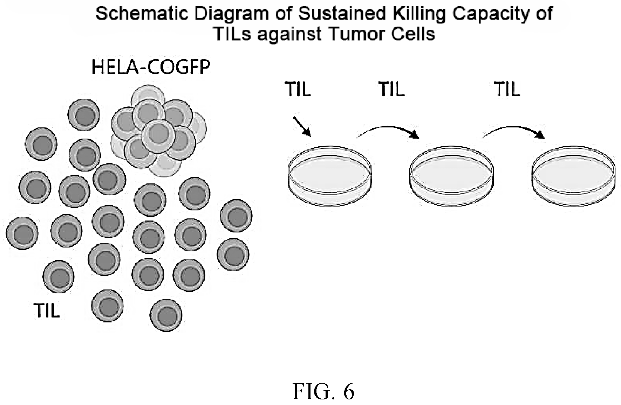

The sustained killing capacity of various TILs against tumor cells was assessed. A schematic diagram of the experiment is shown in Figure 6. The specific procedure was as follows: 1×10⁵ tumor cells HeLa-COGFP (stably expressing COGFP) were seeded into a 24-well plate. After 24 hours, TIL cells were diluted to a density of 1.5×10⁶/mL. The culture medium in the wells of the 24-well plate was aspirated and discarded. The prepared TILs from each group were added at a ratio of 5:1 (TIL:tumor cells). Every 24 hours, 50% of the culture (containing TILs) was transferred to a new 24-well plate containing 1×10⁵ tumor cells HeLa-COGFP per well, and co-culture was continued for another 24 hours. The above procedure was repeated for 3 consecutive times. Each time, the remaining tumor cells in the wells were washed with PBS, and tumor cell viability was determined by CCK8 assay (Meilunbio, MA0218-5) to evaluate the sustained killing of tumor cells by TILs.

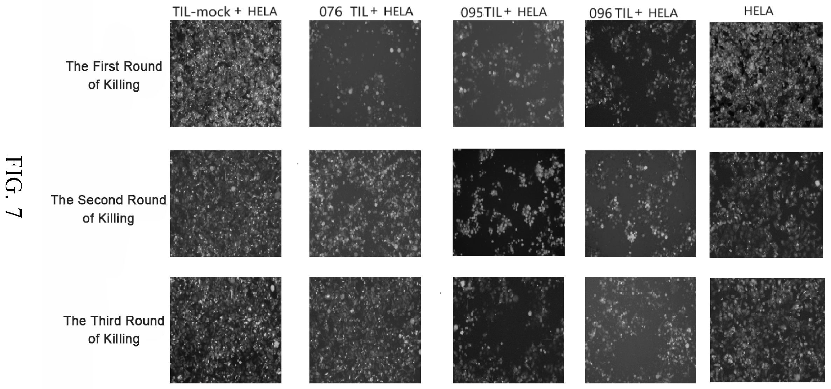

The results of the microscopic images are shown in Figure 7: The HELA cell control group exhibited good cell growth, with cells growing in a dense monolayer. During the first co-culture of HELA cells with 076TIL, 095TIL, and 096TIL, HELA cell growth was slow; the cells failed to completely fill the culture wells, and most cells became rounded and detached. This indicates that 076TIL, 095TIL, and 096TIL all exert a strong cytotoxic effect on HELA tumor cells.

In the second co-culture round, the HELA cells in the HELA+095TIL and HELA+096TIL groups became widely rounded and detached, indicating significant TIL-mediated killing of tumor cells. In contrast, only a small number of HELA cells detached in the 076TIL+HELA group, suggesting that the killing capacity of 076TIL had gradually diminished, whereas the killing capacity of 095TIL and 096TIL showed no significant change compared to the first round.

In the third co-culture round, the HELA cells in the HELA+095TIL and HELA+096TIL groups continued to exhibit widespread rounded and detached, indicating significant TIL-mediated killing of tumor cells. In contrast, the 076TIL+HELA group showed almost no noticeable detachment of HELA cells, suggesting that the killing capacity of 076TIL against HELA cells was significantly reduced, whereas the killing capacity of 095TIL and 096TIL remained at a high level.

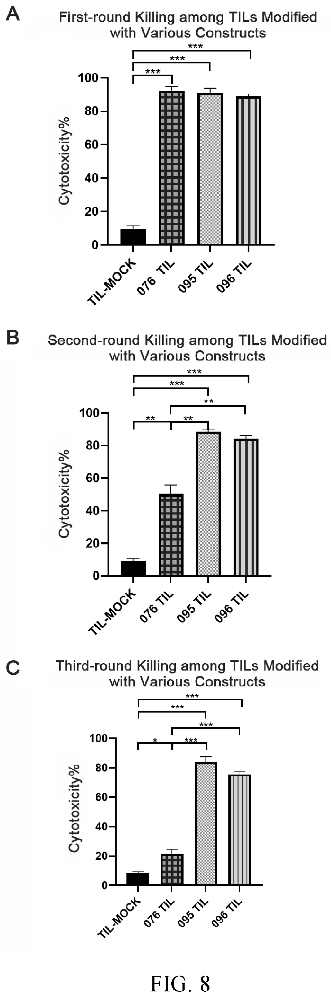

The results of tumor cell activity detection using the CCK8 assay are shown in Figure 8: Compared with 076TIL, 095TIL and 096TIL exhibited higher sustained killing capacity against tumor cells. After three rounds of killing, the cytotoxicity of 076TIL had significantly decreased, whereas 095TIL and 096TIL both maintained high cytotoxicity throughout the three rounds of killing. This indicates that both 095TIL and 096TIL possess excellent sustained killing capacity against tumor cells, with 095TIL exhibiting slightly superior sustained killing capacity compared to 096TIL.

The above results indicate that engineered TIL cells containing the long-acting activation protein of the present invention exhibit high and sustained tumor cell killing capacity.

All documents cited in the present invention are incorporated by reference into this application, as if each were individually cited. Furthermore, it should be understood that, after reading the above description of the present invention, those skilled in the art may make various changes or modifications to the present invention, and such equivalent forms are likewise within the scope defined by the appended claims.

## Claims

1. An engineered TIL cell, wherein the engineered TIL cell expresses an exogenous long-acting activation protein, wherein the long-acting activation protein comprises a DAP10 element and a long-acting activation element; and the sequence of the long-acting activation element is set forth in SEQ ID NO: 2.

2. The engineered TIL cell according to claim 1, wherein the long-acting activation protein comprises, from the N-terminus to the C-terminus, the following structures fused together:
(1) an extracellular domain, comprising a DAP10 element or an active fragment thereof;
(2) a transmembrane domain; and
(3) an intracellular signaling domain, comprising a DAP10 intracellular domain and a long-acting activation element.

3. The engineered TIL cell according to claim 1, wherein the long-acting activation protein has the structure shown in Formula I:
L-ECD-TM-ICD1-ICD2-ICD3 (Formula I)
in Formula I,
"-" is each independently a peptide bond or a linker peptide;
L is absent or a signal peptide;
ECD is an extracellular domain;
TM is a transmembrane domain;
ICD1 is an intracellular domain derived from DAP10 protein;
ICD2 is absent or a co-stimulation domain;
ICD3 is the long-acting activation element.

4. The engineered TIL cell according to claim 3, wherein the ECD is an extracellular domain derived from DAP10 protein.

5. The engineered TIL cell according to claim 3, wherein the ICD2 is one, two or more co-stimulation domain(s) derived from protein(s) selected from the group consisting of: CD40, CD27, 4-1BB, OX40, or a combination thereof.

6. The engineered TIL cell according to claim 1, wherein the amino acid sequence of the long-acting activation protein is set forth in SEQ ID NO: 3 or SEQ ID NO: 4.

7. The engineered TIL cell according to claim 1, wherein the engineered TIL cell comprises a polynucleotide encoding the long-acting activation protein.

8. The engineered TIL cell according to claim 1, wherein the engineered TIL cell comprises a vector comprising a polynucleotide encoding the long-acting activation protein.

9. The engineered TIL cell according to claim 1, wherein the engineered TIL cell further expresses a membrane-bound IL-15 fusion polypeptide, which comprises the following elements fused together:
(i) interleukin 15 (IL-15);
(ii) a transmembrane domain of CD86 and an intracellular domain of CD86.

10. The engineered TIL cell according to claim 1, wherein the engineered TIL cell comprises a nucleic acid construct comprising the polynucleotide encoding the long-acting activation protein.

11. A pharmaceutical composition, wherein the pharmaceutical composition comprises the engineered TIL cell according to claim 1, and a pharmaceutically acceptable carrier

12. A kit, wherein the kit comprises the engineered TIL cell according to claim 1, or an agent used for preparing the engineered TIL cell according to claim 1, wherein the agent is selected from the group consisting of:
(Y1) a polynucleotide encoding the long-acting activation protein; or
(Y2) a vector comprising a polynucleotide encoding the long-acting activation protein.

13. Use of the engineered TIL cell according to claim 1 in the manufacture of a medicament for preventing, alleviating and/or treating a tumor.

14. The use according to claim 13, wherein the tumor comprises: solid tumor, hematologic malignancies, or a combination thereof.

15. A method for treating a disease, wherein the method comprises administering to a subject in need the engineered TIL cell according to claim 1 and/or the pharmaceutical composition according to claim 11.
